(19) Europäisches Patentamt
European Patent Office
Office européen des brevets

(11) EP 1 623 974 A1

(12) **EUROPEAN PATENT APPLICATION**
published in accordance with Art. 158(3) EPC

(43) Date of publication:
**08.02.2006 Bulletin 2006/06**

(21) Application number: **04722438.1**

(22) Date of filing: **22.03.2004**

(51) Int Cl.:
***C07D 205/04** (1985.01)*

(86) International application number:
**PCT/JP2004/003868**

(87) International publication number:
**WO 2004/099133 (18.11.2004 Gazette 2004/47)**

(84) Designated Contracting States:
**AT BE BG CH CY CZ DE DK EE ES FI FR GB GR HU IE IT LI LU MC NL PL PT RO SE SI SK TR**

(30) Priority: **07.05.2003 JP 2003128838**

(71) Applicant: **Japan Science and Technology Agency**
**Kawaguchi-shi,**
**Saitama 332-0012 (JP)**

(72) Inventor: **AOYAGI, Yasuo**
**Hasuda-shi, Saitama 349-0144 (JP)**

(74) Representative: **Herzog, Martin**
**Kahlhöfer . Neumann . Herzog . Fiesser,**
**Karlstrasse 76**
**40210 Düsseldorf (DE)**

# (54) 2"-HYDROXYNICOTIANAMINE, PROCESS FOR PRODUCING THE SAME AND ANGIOTENSIN CONVERTING ENZYME INHIBITOR, HYPOTENSIVE DRUG AND HEALTH FOOD

(57) The invention provides 2"-hydroxynicotianamine; a method for producing 2"-hydroxynicotianamine comprising the step of obtaining an extract solution from a buckwheat sample followed by obtaining a fraction having an inhibitory activity for the angiotensin-converting enzyme by ion-exchange chromatography using an anion exchange resin; novel substances having an ACE inhibitory activity by preparing an inhibitor for the angiotensin-converting enzyme, a vasodepressor agent and a health food each containing 2"-hydroxynicotianamine; methods for producing the novel substances; an inhibitor for the angiotensin-converting enzyme and a vasodepressor agent having the novel substance as an effective ingredient; and a health food containing the novel substance.

EP 1 623 974 A1

## Description

BACKGROUND OF THE INVENTION

Field of the Invention

The invention relates to a novel substance having an ACE inhibitory activity, a method for producing the novel substance or a composition containing the novel substance, and a vasodepressor agent containing the novel substance as an effective ingredient and a health food containing the novel substance.

Description of the Related Art

Since angiotensin-I is converted into angiotensin II having a strong vasopressor function by the action of an angiotensin I converting enzyme (abbreviated as ACE hereinafter) in a renin-angiotensin system that is one of vasopressor systems in the body and is known as an important blood pressure regulation system in human, a substance having an ACE activity suppressing action has been practically used as a vasodepressor agent in the treatment of essential hypertension. However, side effects such as dizziness, postural hypotension, nausea, dry mouth and excessive sedation are currently inevitable.

Nicotianamine extracted from the leaves of tobacco by Noma et al. (M. Noma et al., Tetrahedron Letters, No. 22, pp2017-2020 (1971)) is a natural compound represented by the following formula (2):

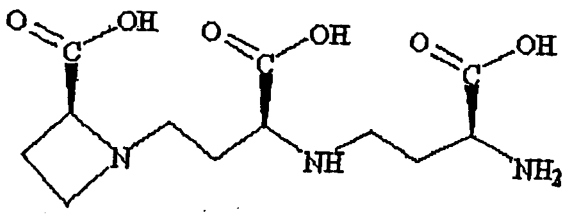

(2)

The compound has an action for suppressing the activity of ACE with less danger of side effects as well as high possibility of vasodepressor effect. Accordingly, a method for producing nicotianamine is described in Japanese Patent Application Laid-Open (JP-A) No. 5-246865 (patent document 1), wherein soybeans are extracted with water or hot water to obtain an extract solution, and nicotianamine is fractionated and collected from the extract solution using a synthetic adsorption resin.

While buckwheat has been considered to be a healthy food with high nutrition values since old times, the protein content in the nutrition component of buckwheat is higher than that in other grains, and its amino acid score is as high as 92 as compared with 65 in polished rice and 41 in wheat flour. The quality of the protein contained in buckwheat is excellent. In addition, while buckwheat contains more dietary fibers than other grains, it is reported that the buckwheat protein also has a dietary fiber-like action and inhibits absorption of cholesterols. While rutin has been known since old time as one of functional components of buckwheat that reinforces capillary vessels by a synergic action with vitamin C, many polyphenols including rutin have been isolated according to recent research results, and their functions based on an antioxidative effect have been noticed.

In relation of buckwheat with the renin-angiotensin system, a hot water extract and protein degradation products of buckwheat have been reported to have ACE inhibitory ability, and vasodepressor action of buckwheat has been considered to be derived from rutin and peptides.

An extract solution of threshed seeds of buckwheat or buckwheat flour with water or hot water is fractionated by gel filtration column chromatography, the fractions having the ACE inhibitory activity are collected, and each fraction obtained is fractionated by adsorption column chromatography to collect fractions having a strong ACE inhibitory activity. These fractions are further fractionated by gel filtration column chromatography to obtain fractions having a stronger ACE inhibitory activity. Consequently, a novel natural product represented by the following formula (3), which has an ACE inhibitory activity and high possibility to exhibit vasodepressor action, has been obtained, as described in Japanese Patent Application Laid-Open (JP-A) No. 5-97798 (patent document 2):

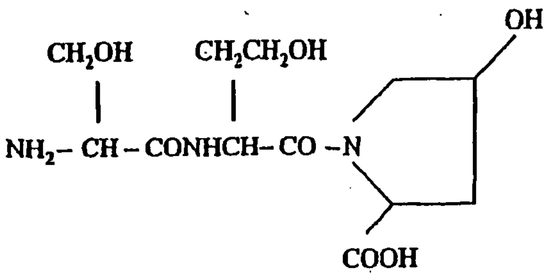

(3)

While these natural compounds having the ACE inhibitory activity are quite likely to be used for a vasodepressor agent and health food, researches on novel substances having the ACE inhibitory activity cannot be considered to be sufficient, and additional survey of the novel substance having the ACE inhibitory activity is required.

SUMMARY OF THE INVENTION

Accordingly, the object of the invention is to provide a novel substance having an ACE inhibitory activity, a method for producing the novel substance or a composition containing the novel substance, an angiotensin I-converting enzyme inhibitor and a vasodepressor agent containing the novel substance as an effective ingredient, and a health food containing the novel substance.

The inventors of the invention have completed the invention by finding, through intensive studies, that the substance obtained by the following steps is a novel substance having the ACE inhibitory activity that is 2"-hydroxynicotianamine. The process for obtaining the novel substance comprises the steps of: extracting a buckwheat sample such as buckwheat flour, unpolished buckwheat grains, stems and leaves of buckwheat, buds of buckwheat, Tartary buckwheat flour, unpolished Tartary buckwheat grains, stems and leaves of Tartary buckwheat and hot water after boiling buckwheat noodles with an extraction solvent to obtain an extract solution; and separating and purifying the extract solution by ion-exchange chromatography using an anion exchange resin to obtain a fraction containing the ACE inhibitory activity.

In a first aspect, the invention provides 2"-hydroxynicotianamine represented by the following formula (1) :

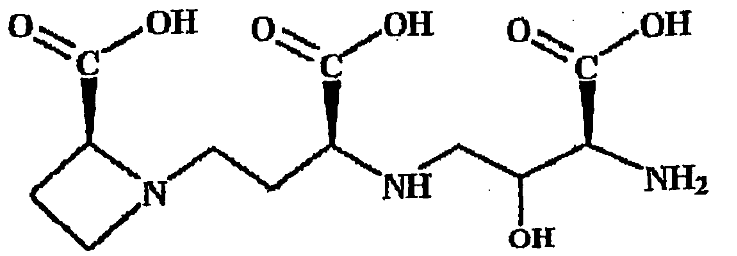

(1)

The invention as disclosed in the first aspect is able to manifest an effect for providing a novel substance having an ACE inhibitory activity.

In a second aspect, the invention provides a method for producing 2"-hydroxynicotianamine comprising the steps of: extracting a buckwheat sample with an extraction solvent to obtain an extract solution; and separating and purifying the extract solution by ion-exchange chromatography using an anion exchange resin to obtain a fraction having an angiotensin-converting enzyme inhibitory activity. The invention as disclosed in the second aspect is able to manifest an effect for producing 2"-hydroxynicotianamine having the ACE inhibitory activity.

In a third aspect, the invention provides a method for producing 2"-hydroxynicotianamine according to the second aspect, wherein the extraction solvent is an aqueous ethanol solution. The invention as disclosed in the third aspect is able to manifest an effect for improving extraction performance in the extraction process as well as the effect disclosed in the second aspect.

In a fourth aspect, the invention provides a method for producing 2"-hydroxynicotianamine according to the second or third aspect comprising the steps of: obtaining concentrated solution by concentrating the extract solution; and subjecting the concentrated solution to separation and purification. The invention as disclosed in the fourth aspect is able to manifest an effect for reducing the sample volume in the separation and purification process as well as the effects disclosed in the second and third aspects.

In a fifth aspect, the invention provides a method for producing 2"-hydroxynicotianamine according to any one

of the second to fourth aspects comprising the step of applying the separation and purification process twice or more. The invention as disclosed in the fifth aspect is able to manifest an effect for reducing impurities contained in the product obtained as well as the effect disclosed in any one of the second to fourth aspects.

In a sixth aspect, the invention provides a method for producing 2''-hydroxynicotianamine according to any one of the second to fifth aspects comprising the steps of: subjecting the extract solution or concentrated solution to ion-exchange chromatography using a cation exchange resin to obtain a retention product retained on the cation exchange resin, followed by obtaining an eluate by eluting the retention product; and separating and purifying the eluate. The invention as disclosed in the sixth aspect is able to manifest an effect for reducing the amount of impurities contained in the product obtained by removing substances not retained on the cation exchange resin, as well as the effect disclosed in any one of the second to fifth aspects.

In a seventh aspect, the invention provides a method for producing 2''-hydroxynicotianamine according to any one of the second to sixth aspects comprising the step of obtaining an aqueous layer as the extract solution or concentrated solution by extracting once or at least twice the extract solution or concentrated solution obtained by the extraction or concentration process with one or at least two kinds of organic solvents. The invention as disclosed in the seventh aspect is able to manifest an effect for reducing impurities contained in the product obtained by defatting, as well as the effect disclosed in any one of the second to sixth aspects.

In a eighth aspect, the invention provides a method for producing 2''-hydroxynicotianamine according to any one of the second to seventh aspects comprising the step of isolating crystals by recrystallization of a fraction having the angiotensin-converting enzyme inhibitory activity after applying the separation and purification process. The invention as disclosed in the eighth aspect is able to manifest an effect for obtaining high purity 2''-hydroxynicotianamine as well as the effect disclosed in any one of the second to seventh aspects.

In a ninth aspect, the invention provides a method for producing 2''-hydroxynicotianamine according to the eighth aspect, wherein the solvent used for recrystallization in the isolation step is an aqueous ethanol solution. The invention as disclosed in the ninth aspect is able to manifest an effect for increasing the amount of 2''-hydroxynicotianamine obtained in recrystallization, as well as the effect disclosed in the eighth aspect.

In the tenth aspect, the invention provides an inhibitor for the angiotensin-converting enzyme containing 2''-hydroxynicotianamine according to the first aspect as an effective ingredient.

In the eleventh aspect, the invention provides a vasodepressor agent containing 2''-hydroxynicotianamine according to the first aspect as an effective ingredient.

In a twelfth aspect, the invention provides a health food containing 2''-hydroxynicotianamine according to the first aspect.

BRIEF DESCRIPTION OF THE DRAWINGS

Fig. 1 shows $^1$H-NMR spectrum of the novel substance according to the invention;
Fig. 2 shows $^{13}$C-NMR spectrum of the novel substance according to the invention;
Fig. 3 shows $^1$H-$^1$H COSY spectrum of the novel substance according to the invention;
Fig. 4 shows $^{13}$C-$^1$H COSY spectrum of the novel substance according to the invention; and
Fig. 5 shows a positive product ion spectrum of the novel substance of the invention by m/z320$(M+H)^+$ in FAB (Fast Atom Bombardment)-MS.

DESCRIPTION OF THE PREFERRED EMBODIMENTS

While embodiments of the invention are described hereinafter, the invention is by no means restricted to these embodiments. 2''-Hydroxynicotianamine (referred to a novel substance hereinafter) of the invention has following chemical and physical properties.

<$^1$H-NMR>

The spectrum is measured in heavy water using TMSP as an internal standard at a frequency of 270 MHz. Multiplicity, coupling constant and assignment are shown in the parenthesis.

The novel substance of the invention has $^1$H-NMR peaks at 52.05 to 2.3 ppm (2H, m, H-2'), 52.4 to 2.85 ppm (2H, m, H-3), 53.25 to 3.53 ppm (2H, m, H-1'' and H-1'), 53.85 ppm (1H, dd, J=4.78 Hz, J=8.42 Hz, H-3'), 53.92 to 4.15 ppm (2H, m, H-4), 54.01 ppm (1H, d, J=3.3 Hz, H-3''), 54.42 to 4.48 ppm (1H, m, H-2'') and δ4.77 ppm (1H, t, J=9.57Hz, H-2). The $^1$H-NMR spectrum is shown in Fig. 1.

<$^{13}$C-NMR>

The spectrum is measured in heavy water using TMSP as an internal standard at a frequency of 68 MHz. Assignment is shown in the parenthesis.

The novel substance of the invention has $^{13}$C-NMR peaks at 523.9 ppm (C-3), 527.6 ppm (C-2'), 551.2 ppm (C-1"), 553.4 ppm (C-4), δ54.1 ppm (C-1'), 560.5 ppm (C-3"), 562.4 ppm (C-3'), 568.3 ppm (C-2"), 569.7 ppm (C-2), 5173.3 ppm (C-4"), 5174.9 ppm (C-4') and 5176.0 ppm (C-1). The $^{13}$C-NMR spectrum is shown in Fig. 2.

<$^{1}$H-$^{1}$H COSY and $^{13}$C-$^{1}$H COSY>

In the novel substance of the invention, the $^{1}$H-$^{1}$H COSY spectrum shown in Fig. 3 is obtained by $^{1}$H-$^{1}$H COSY, and the $^{13}$C-$^{1}$H COSY spectrum shown in Fig. 4 is obtained by $^{13}$C-$^{1}$H COSY.

<MS>

A weak (M+H) peak was observed at m/z=320 in FAB-MS when the novel substance of the invention was measured by FAB (Fast Atom Bombardment)-MS and ESI (Electrospray Ionization)-MS. The peak at m/z=320 was also confirmed to be (M+H) peak in ESI-MS.

<High resolution MS>

The peak of (M+H) ions was appeared at m/z=320.1458 in high resolution FAB-MS, which is consistent with the mass number 320.1458 calculated from $C_{12}H_{22}O_7N_3$, and the molecular formula was confirmed to be $C_{12}H_{21}O_7N_3$.

<Other chemical and physical properties>

While the novel substance of the invention is positive to the ninhydrin reaction, the reaction is suppressed by $Cu^{2+}$ ions. The novel substance of the invention is soluble in water, dilute acid and dilute alkali. Almost no usual amino acids are detected when amino acids are detected with an automatic amino acid analyzer after decomposing the novel substance of the invention with 6N HCl at 110°C for 16 hours, and the decomposition product has a complicated composition. The novel substance of the invention has a melting point of 275 to 280°C and a specific rotation $[\alpha]_D^{23.6}$ of -40° (c=0.4 g/100ml; solvent = water) and $[\alpha]_D^{23.6}$ of -34.5° (c=0.2 g/100ml/; solvent = 1N HCl) The novel substance showed single spot at Rf of 0.03 and 0.06 in thin layer chromatography (TLC) by means of silica gel support using a mixed development solvents of n-butanol: acetic acid : water - 4:1:2 and n-propanol : ammonia = 7:3, respectively.

The novel substance of the invention is proved to have a molecular weight of 319 from the spectrum obtained by FAB-MS and ESI-MS, and is proved to be represented by a molecular formula of $C_{12}H_{21}O_7N_3$ obtained by high resolution MS. The substance is presumed to be an amino acid or peptide since it is positive to the ninhydrin reaction. However, the substance was presumed to be a non-protein amino acid, not a peptide, since the substance yielded a complicated decomposition product in the process for detecting amino acids, and was presumed to be an α-amino acid since the ninhydrin reaction is suppressed by $Cu^{2+}$ ions. From the results of analysis based on the analysis of spectra obtained by $^{1}$H-NMR, $^{13}$C-NMR, $^{1}$H-$^{1}$H COSY and $^{13}$C-$^{1}$H COSY, and from total analysis of various data above, the substance of the invention was presumed to be a 2'- or 2"-hydroxy derivative of nicotianamine. A peak at m/z=56 assigned to an azetidine ring was observed in addition to peaks at m/z=114, 128, 186 and 201 assigned to those of nicotianamine in a product ion spectrum (Fig. 5) derived from positive ions at m/z=320 (M+H) in FAB-MS. Peaks at m/z=245 and 191 show the presence of a hydroxyl group at 2"-position. The structure of the novel substance of the invention was determined to be 2"-hydroxynicotianamine from these data.

<Buckwheat sample>

The method for producing the novel substance of the invention is described below. While any materials containing the novel substance of the invention including natural non-processed samples derived from buckwheat as a plant as well as processed materials such as processed foods using buckwheat may be used as buckwheat samples, examples of the non-processed natural samples available include buckwheat flour, unpolished buckwheat grains, stems and leaves of buckwheat, buds of buckwheat and Tartary buckwheat, and examples of the processed material include hot water after boiling buckwheat noodles. Buckwheat flour may be favorably used among them. While these buckwheat samples may be directly used for the extraction process, it may be used in the extraction process after pulverizing.

<Extraction solvent>

While the extraction process in the method for producing the novel substance of the invention is to obtain an extract solution by extracting the buckwheat sample with an extraction solvent, examples of the solvent include water, hot water, an aqueous ethanol solution, an aqueous methanol solution and an aqueous dilute acid solution. The aqueous ethanol solution is preferable, and the aqueous ethanol solution preferably has an ethanol concentration of 60 to 80% by volume.

<Procedure of extraction process>

An extract solution is obtained by extracting the buckwheat sample with the extract solvent as described above in the extraction process. While the extraction method is not particularly restricted and any method known in the art may be used for extraction, the methods include extracting the buckwheat sample by mixing it with the extract solvent followed by filtration to obtain a filtrate as the extract solution, and extracting a plant sample by mixing with the extraction solvent followed by centrifugation, and obtaining the supernatant as the extract solution. While the temperature of the extraction solvent for extraction is not particularly restricted, a temperature of 50 to 100°C is preferable, for example, for the aqueous ethanol solution since extractability of the solvent is excellent in this temperature range. While the higher volume ratio of the extraction solvent relative to 1 kg of the buckwheat sample enhances extraction efficiency, the ratio is preferably about 10 times by volume. While the extraction condition and times may be appropriately selected depending on the samples, operations for obtaining an extract solution by adding the extraction solvent may be repeated several times. Extraction with one or plural kinds of organic solvents may be applied once or at least twice to obtain an aqueous layer as the extract solution after the extraction process as described above, wherein the extract solution obtained by extraction with the aqueous ethanol solution is further extracted with hexane, ethyl acetate or diethylether, and the aqueous layer is used as the extract solution. Examples of the organic solvent available for this object include hexane, ethyl acetate, diethylether and petroleum ether.

<Concentration process>

In the method for producing the novel substance of the invention, a concentrated solution may be subjected to a separation and purification process below after a concentration process for obtaining the concentrated solution by concentrating the extract solution obtained in the extraction process above. The extract solution is concentrated using concentration means such as a rotary evaporator to obtain the concentrated solution in the concentration process. Alternatively, the extract solution may be extracted with one or at least two kinds of organic solvents to obtain the aqueous concentrated solution. The methods include extracting concentrated solution with diethylether or hexane leaving an aqueous layer, and the aqueous layer is further extracted with ethyl acetate to obtain the aqueous layer as the concentrated solution. The organic solvents listed in "Procedure of Extraction Process" may be used as the organic solvent.

<Elution process>

In the method for producing the novel substance of the invention, a retention product retained on the cation exchange resin is obtained by subjecting the extract solution obtained in the extraction process or the concentrated solution obtained in the concentration process to ion-exchange chromatography using a cation exchange resin. The retained product is eluted in an elution step, and the eluate is subjected to a separation and purification process described blow. The retention product retained on the cation exchange resin is preferably adsorbed on the cation exchange resin.

<Column resin used in elution process>

While the cation exchange resin filled in a column in ion-exchange chromatography using the cation exchange resin is not particularly restricted, the resin is preferably a strong cation exchange resin such as Amberlite IR-120 (manufactured by Rohm and Haas Co.) or Dowex 50 (manufactured by Dow Chemical Co.).

<Elution agent for elution>

An example of the eluant is an aqueous solution of an electrolyte solution such as ammonia, pyridine, sodium hydroxide and potassium hydroxide. Ammonia is particularly preferable among these electrolytes. The concentration of the electrolyte in the eluant is preferably 0.5 to 2.0 mol/L.

<Procedure of elution process>

The elution process comprises the steps of: injecting the extract solution obtained through the extraction process or the concentrated solution obtained in the concentration process into a column filled with the cation exchange resin by a known method such as a syringe method or valve loop method; obtaining a retention product by allowing positively charged substances contained in the extract solution or concentrated solution to be retained on the column; and eluting the retained product by flowing an elution agent through the column to obtain an eluate. The eluate is measured for the ACE inhibitory activity, and a fraction having the ACE inhibitory activity of the plural fractions of the eluate may be collected as the eluate. The fraction having the ACE inhibitory activity may be isolated by subjecting the eluate thus obtained to the separation and purification process below.

<Separation and purification process>

Since the novel substance contained in the extract solution, concentrated solution or eluate has a property to be retained or adsorbed on the cation exchange resin, it was proved to be positively charged. In the separation and purification process of the method for producing the novel substance of the invention, the positively charged novel substance of the invention contained in the extract solution, concentrated solution or eluate is separated and purified by ion-exchange chromatography using the anion exchange resin to obtain a fraction having an angiotensin I-converting enzyme inhibitory activity.

<Column resin>

While the anion exchange resin filled in the column in ion-exchange chromatography using the anion exchange resin is not particularly restricted, a strong anion exchange resin such as Dowex 1 (manufactured by Dow Chemical Co.) is preferable. The anion exchange resin is used as an acetic acid type resin.

<Eluant in separation and purification process>

Examples of the eluant include aqueous solutions of electrolytes such as acetic acid, formic acid, hydrochloric acid, ammonia and pyridine.

<Procedure of separation and purification process>

The separation and purification process comprises the steps of: injecting the extract solution obtained in the extraction process into a column filled with the cation exchange resin or anion exchange resin by a known method; washing the column with water and eluting the column with the aqueous electrolyte solution as the eluant; and measuring the ACE inhibitory activity of the fractions obtained to collect the fractions having the ACE inhibitory activity. Known methods such as the method of Cushman and Cheung may be used for measuring the ACE inhibitory activity.

<Isolation process>

A powder of 2"-hydroxynicotianamine of the invention can be obtained from the fraction having the ACE inhibitory activity by known methods for obtaining a dried powder substance by subjecting the fraction having the ACE inhibitory activity obtained by the procedure above to known drying means such as a rotary evaporator, spray dryer and freeze dryer. However, the powder of the novel substance obtained from the fraction having the ACE inhibitory activity is preferably obtained as a colorless and crystalline novel substance by an isolation process comprising the steps of recrystallization and isolation of the crystals. While examples of solvents for recrystallization include an aqueous acetone solution, an aqueous methanol solution, water, and an aqueous ethanol solution, the aqueous ethanol solution is preferable for facilitating recrystallization. The ethanol concentration in the aqueous ethanol solution is 10 to 80% by volume, preferably 30 to 60% by volume, relative to 100% by weight of the aqueous ethanol solution. While the temperature for recrystallization from the aqueous ethanol solution is not particularly restricted, the powder of 2"-hydroxynicotianamine is dissolved in the solvent at 70 to 100°C, and 2"-hydroxynicotianamine is recrystallized at -10 to 10°C.

The novel substance obtained in the method for producing the novel substance of the invention as described above has the chemical and physical properties as described in <$^1$H-NMR>, <$^{13}$C-NMR>, <$^1$H-$^1$H COSY and $^{13}$C-$^1$H COSY>, <MS>,

<High Resolution MS> and <Other Chemical and Physical Properties>.

2"-Hydroxynicotianamine of the invention may be formulated into the effective ingredient of the ACE inhibitory agent. 2"-Hydroxynicotianamine of the invention may be formulated into the ACE inhibitory agent in the form of tablets, capsules, troche, powder or fine granules, granules and aqueous solution by conventional means using a pharmaceutically acceptable carrier such as excipient, humectant, binder and flavoring agent together. The ACE inhibitory agent can be used as a vasodepressor agent. The ACE inhibitory agent or vasodepressor agent may be orally administered or intravenously administered as an injection agent.

Breads, cakes, biscuits,cookies, candies, noodles such as buckwheat noodles, wheat noodles and Chinese noodles, and daily dishes such as tempura and fries may be processed into health foods by adding 2"-hydroxynicotianamine of the invention. The health foods may be also provided by dissolving and adding 2"-hydroxynicotianamine of the invention in juice, coffee, cocoa, oolong tea and green tea.

While the amount of addition of 2"-hydroxynicotianamine of the invention in the health food is not particularly restricted, the amount is preferably 10 mg or more/100 g for the foods such as bread, candy, noodle and daily dishes, and 5 mg or more/100 g for beverages. 2"-Hydroxynicotianamine of the invention may be directly used for the health food and pharmaceuticals.

(Examples)

While the invention is described in detail below with reference to examples, the descriptions are only provided as examples, and are not intended to restrict the invention.

Example 1

<Extraction process>

Added in 2 Kg of buckwheat flour was 8 liters of aqueous 70% ethanol solution, and the flour was extracted by allowing it to stand at room temperature overnight. The extract was filtered with filter paper to obtain a first extract solution. The residue was extracted again with the same volume of 70% aqueous ethanol solution, and the extracted solution was filtered with filter paper. This filtration steps were repeated twice to obtain a second and third extract solution.

<Concentration process>

The first, second and third extract solution were combined into an extract solution, which was concentrated to about 2 liters using a rotary evaporator.

The concentrated solution was extracted with hexane using a separation funnel to obtain a hexane layer and aqueous layer 1. When the ACE inhibitory activity was measured for the hexane layer and aqueous layer 1 according to <Measurement of ACE Inhibitory activity> described below, the ACE inhibitory activity was found in aqueous layer 1 but not in the hexane layer, and indicated that the activity remained in aqueous layer 1. Subsequently, aqueous layer 1 was extracted with ethyl acetate to obtain an ethyl acetate layer and aqueous layer 2. When the ACE inhibitory activity was measured for the ethyl acetate layer and aqueous layer 2 according to <Measurement of ACE Inhibitory activity> described below, the ACE inhibitory activity was found in aqueous layer 2 but not in the ethyl acetate layer, and indicated that the activity remained in aqueous layer 2. Aqueous layer 2 was used as a concentrated solution and was used for the separation and purification process described below.

<Elution process>

Cation exchange resin Amberlite IR-120($H^+$) was filled in a column, thoroughly washed with water, and was equilibrated. An aqueous ammonia solution with a concentration of 1 mol/L was prepared as an elution solution. The concentrated solution containing an inhibitory activity was directly injected into the column filled with cation exchange resin Amberlite IR-120($H^+$), and non-adsorbed fractions were collected by washing the column with water. Adsorbed fractions were eluted with an aqueous $NH_4OH$ solution with a concentration of 1 mol/L as an eluant to obtain an eluate. Each fraction was concentrated to dryness in the rotary evaporator, and the residue was dissolved in a given volume of water. The ACE inhibitory activity of the aqueous solution was measured according to <Measurement of ACE Inhibitory Activity> described below, and found that the activity remained only in the eluate. This shows that the active component is a positively charged substance.

<Separation and purification process>

Anion exchange resin Dowex 1 x 4 was filled in a column, thoroughly washed with water, and equilibrated. An aqueous acetic acid solution with a concentration of 0.3 mol/L was prepared as an eluant. The eluant was adjusted to pH 8 using an aqueous ammonia, and injected into the column of anion exchange resin Dowex 1 x 4. Pure water, and then the eluant were flowed through the column to obtain a plurality of fractions. The plural fractions were measured for the ACE inhibitory activity according to <Measurement of ACE Inhibitory Activity> described below, and a fraction having the ACE inhibitory activity was obtained. Subsequently, the fraction having the ACE inhibitory activity was subjected to ion-exchange chromatography twice using the column of anion exchange resin Dowex 1 x 4 to purify the fraction having the ACE inhibitory activity. Consequently, a fraction containing a substance having a strong ACE inhibitory activity was obtained, and 85 mg of the substance having a strong ACE inhibitory activity was obtained after concentrating the fraction to dryness using a rotary evaporator.

<Isolation process>

The substance having a strong ACE inhibitory activity (85 mg) was dissolved in a mixed solution of water-ethanol (ethanol content 40% by volume), and was recrystallized twice from the solution to isolate 44 mg of colorless crystals. The spectra shown in Figs. 1 to 5 were obtained by the measurements of $^1$H-NMR, $^{13}$C-NMR, $^1$H-$^1$H COSY, $^{13}$H-$^1$C COSY, MS and high resolution MS, respectively, of the isolated colorless crystal. The colorless crystal was proved to be a novel substance having the chemical and physical properties described in <Other Chemical and Physical Properties>. The novel substance is considered to be 2"-hydroxynicotianamine.

<Measurement of ACE inhibitory activity>

The ACE inhibitory activity was measured as follows by Cushman and Cheung method.

(1) Preparation of reagents

(i) Added in 100 ml of water were 1.60 mg, 0.8 mg and 0.4 mg of the novel substances of the invention, or the colorless crystal, and sample solutions 1, 2 and 3 with the concentrations of the novel substance of the invention of 50 μmol/L, 25 μmol/L and 12.5 μmol/L, respectively, were prepared.
(ii) A buffer solution (pH 8.3) with a boric acid concentration of 0.2 mol/L was prepared.
(iii) Hippuryl-L-histidyl-L-leucine was dissolved in the buffer solution at a concentration of 7 mmol/L to prepare a substrate solution.
(iv) An aqueous NaCl solution with a concentration of 2 mol/L was prepared.
(v) ACE used was a freeze dry product of ACE from the lung of rabbit (manufactured by Sigma), and an enzyme solution with an ACE activity of 150 mIU/ml was prepared by adding 1 IU of the ACE to 6.67 ml of the buffer solution.

(2) Composition of reaction solution

(i) substrate solution: 500 μl,
(ii) 2 mol/L NaCl solution: 400 μl,
(iii) sample solutions 1 to 3: 30 μl each,
(iv) water: 40 μl,
(v) enzyme solution: 30 μl

(3) Reaction condition

After mixing the compositions of the reaction solution (i), (ii), (iii), and (iv), enzyme solution (v) was added to the mixed solution without pre-incubation, and the solution was allowed to react (incubate) at 37°C for 30 minutes. Then, 500 μl of 1 mol/L hydrochloric acid was added to stop the reaction. Free hippuric acid was extracted with 3 ml of ethyl acetate. The extract solution was evaporated to dryness using a centrifugal evaporator, and the residue was dissolved in 1 ml of water to measure the concentration of the extract by absorbance at 228 nm using a spectrophotometer. The measured absorbance was defined as ODs (ODs1, ODs2 and ODs3) for the measurements using samples solutions 1,

2 and 3, respectively.

(4) Measurement of control

The reaction was performed under the same condition as in (3) above, except that water (30 μl) was used in place of sample solutions (iii) in the reaction condition (3) above. The absorbance obtained at 228 nm was defined as ODc.

(5) Measurement of blank

Compositions (i) to (iv) of the reaction solution were mixed, and allowed to react at 37°C for 30 minutes. After stopping the reaction by adding 1 mol/L of HCl, enzyme solution (v) was added. The mixed solution was extracted with ethyl acetate as in (3), and the same procedure as in (3) was applied to the extract solution. The absorbance obtained at 228 nm was defined as ODb.

(6) Determination of the concentration of the novel substance of the invention that indicates 50% inhibition (IC50) of ACE

The rates of inhibition of ACE were determined by the following equation (4) using ODs1, ODs2 and ODs3:

$$\text{Rate of inhibition of ACE}(\%) = [1 - (ODs - ODb)/(ODc - ODb)] \times 100 \qquad (4)$$

The concentration of the novel substance of the invention that indicates 50% inhibition of ACE was determined by extrapolation on the graph. The result showed that the concentration of the novel compound of the invention that indicates 50% inhibition (IC50) of ACE was 0.08 μmol/L.

<u>Comparative Example 1</u>

The measurement described in <Measurement of ACE Inhibitory Activity> was performed by the same method as in Example 1, except that sample solutions 4, 5 and 6 with nicotianamine concentrations of 50 μmol/L, 25 μmol/L and 12.5 μmol/L, respectively, were prepared by adding 1.51 mg, 0.76 mg and 0.38 mg, respectively, of nicotianamine in 100ml of water in preparation of the reagent described in (1), and the solutions were used as the sample solutions (iii) of the composition of the reaction solution in (2) under the reaction condition in (3). The results showed that the concentration of nicotianamine that shows 50% inhibition (IC50) of ACE activity was 0.085 μmol/L from equation (4) using the absorbance ODs of sample solutions 4 to 6 (ODs4, ODs5 and ODs6), and ODc and ODb determined in Example 1.

The results in Example 1 and Comparative Example 1 showed that IC50 of the ACE inhibitory activity of the novel substance (2"-hydroxynicotianamine) of the invention was 0.08 μM, which indicates approximately the same or stronger inhibitory activity as compared with IC50 of 0.085 μm of nicotianamine measured under the same condition.

2"-Hydroxynicotianamine of the invention has an ACE inhibitory activity. 2"-hydroxynicotianamine of the invention having the ACE inhibitory activity can be produced by the method for producing the novel substance of the invention. An inhibitor for inhibiting the angiotensin I-converting enzyme and a vasodepressor agent can be formulated adding 2"-hydroxynicotianamine of the invention as an effective ingredient, and a health food can be prepared by adding 2"-hydroxynicotianamine of the invention.

## Claims

1. 2"-hydroxynicotianamine represented by the following formula (1):

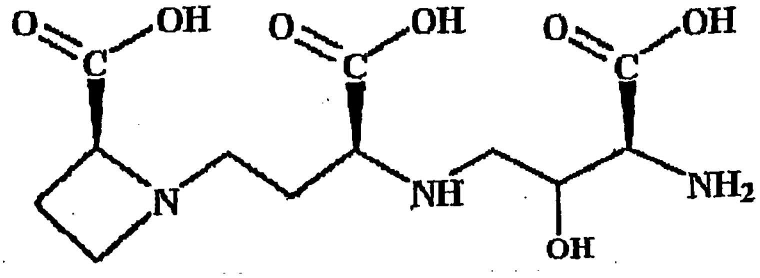

(1)

2. A method for producing 2"-hydroxynicotianamine comprising the steps of: extracting a buckwheat sample with an extraction solvent to obtain an extract solution; and separating and purifying the extract solution by ion-exchange chromatography using an anion exchange resin to obtain a fraction having an angiotensin I-converting enzyme inhibitory activity.

3. A method for producing 2"-hydroxynicotianamine according to Claim 2, wherein the extraction solvent is an aqueous ethanol solution.

4. The method for producing 2"-hydroxynicotianamine according to Claim 2 or 3 comprising the steps of: obtaining concentrated solution by concentrating the extract solution; and subjecting the concentrated extract to separation and purification.

5. The method for producing 2"-hydroxynicotianamine according to any one of Claims 2 to 4 comprising the step of applying the separation and purification process twice or more.

6. The method for producing 2"-hydroxynicotianamine according to any one of Claims 2 to 5 comprising the steps of: subjecting the extract solution or concentrated solution to ion-exchange chromatography using a cation exchange resin to obtain a retention product retained on the cation exchange resin, followed by obtaining an eluate by eluting the retained product; and separating and purifying the eluate.

7. The method for producing 2"-hydroxynicotianamine according to any one of Claims 2 to 6 comprising the step of obtaining an aqueous layer as the extract solution or concentrated solution by extracting once or at least twice the extract solution or concentrated solution obtained by the extraction or concentration process with one or at least two kinds of organic solvents.

8. The method for producing 2"-hydroxynicotianamine according to any one of Claims 2 to 7 comprising the step of isolating crystals by recrystallization of a fraction having an activity for inhibiting an angiotensin-converting enzyme activity after the separation and purification process.

9. The method for producing 2"-hydroxynicotianamine according to Claim 8, wherein the solvent used for recrystallization in the isolation step is an aqueous ethanol solution.

10. An inhibitor for the angiotensin-converting enzyme containing 2"-hydroxynicotianamine according to Claim 1 as an effective ingredient.

11. A vasodepressor agent containing 2"-hydroxynicotianamine according to Claim 1 as an effective ingredient.

12. A health food containing 2"-hydroxynicotianamine according to Claim 1.

Fig 1

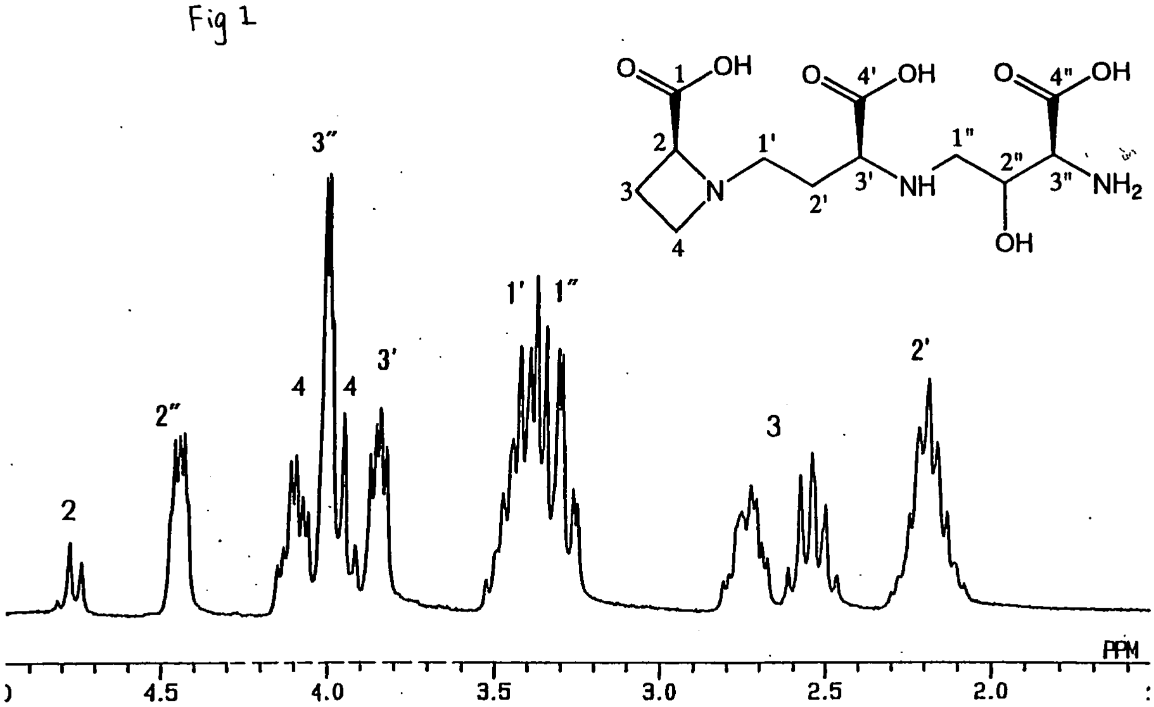


Fig2 Assignment

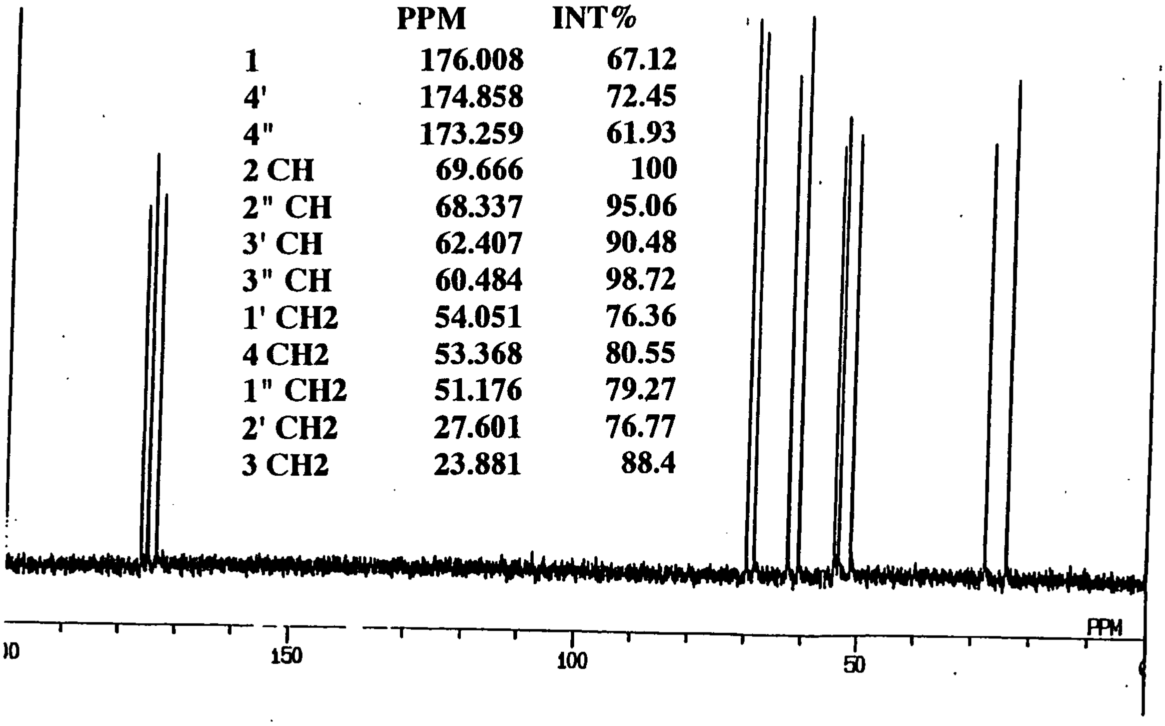


| | PPM | INT% |
|---|---|---|
| 1 | 176.008 | 67.12 |
| 4' | 174.858 | 72.45 |
| 4" | 173.259 | 61.93 |
| 2 CH | 69.666 | 100 |
| 2" CH | 68.337 | 95.06 |
| 3' CH | 62.407 | 90.48 |
| 3" CH | 60.484 | 98.72 |
| 1' CH2 | 54.051 | 76.36 |
| 4 CH2 | 53.368 | 80.55 |
| 1" CH2 | 51.176 | 79.27 |
| 2' CH2 | 27.601 | 76.77 |
| 3 CH2 | 23.881 | 88.4 |

Fig 3

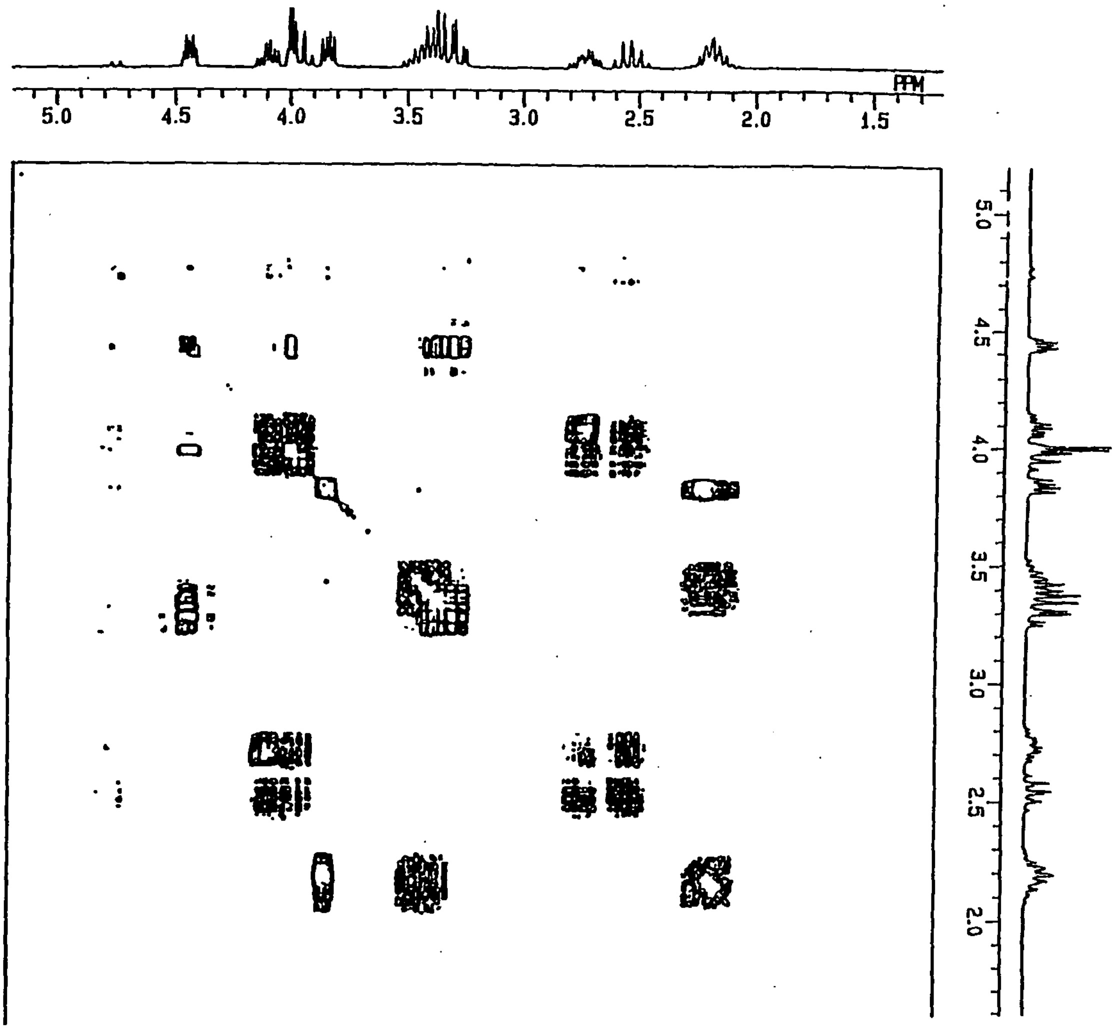

Fig 4

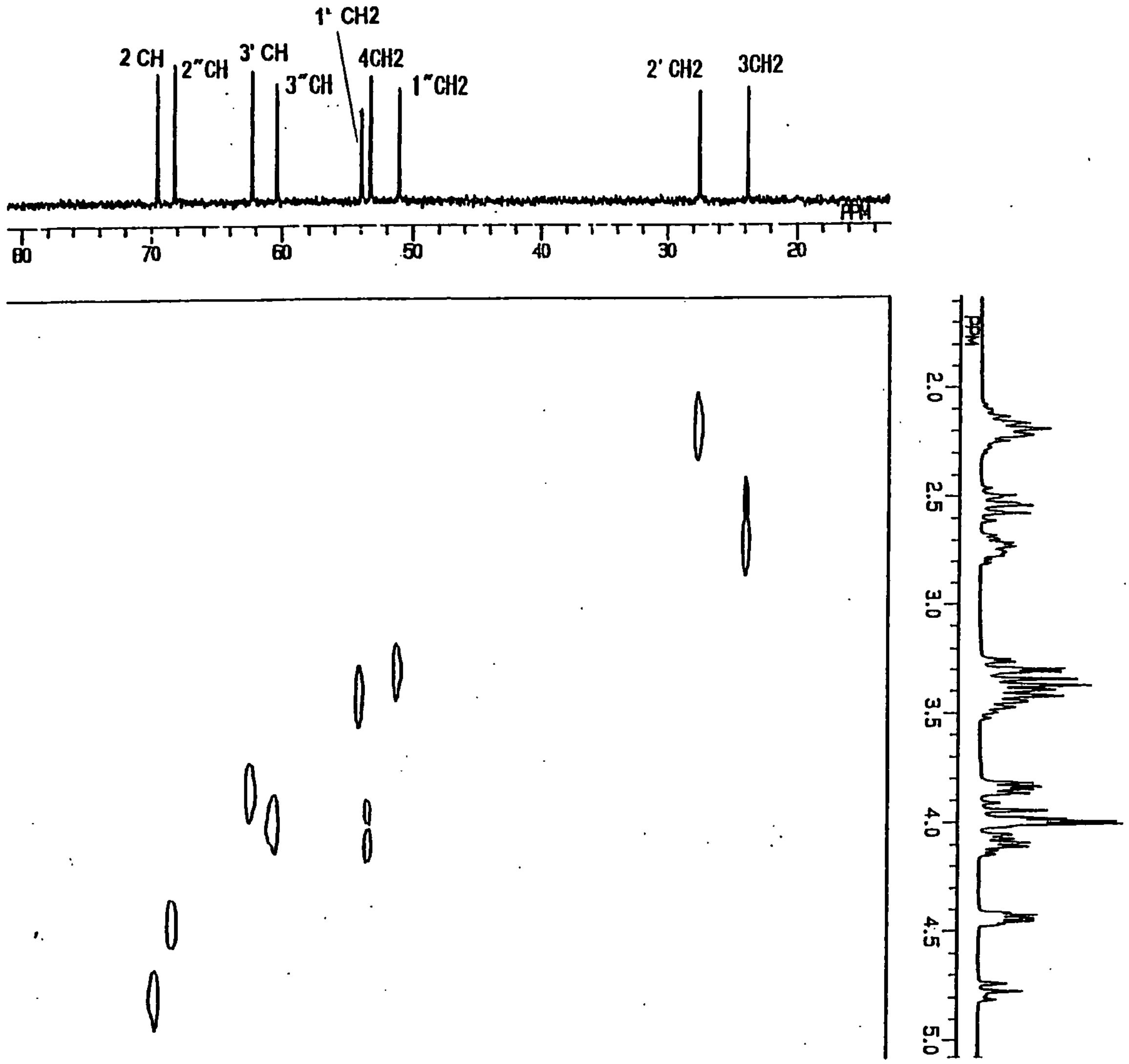
1' CH2
2 CH
2"CH
3' CH
3"CH
4CH2
1"CH2
2' CH2
3CH2
PPM
80
70
60
50
40
30
20
PPM
2.0
2.5
3.0
3.5
4.0
4.5
5.0

Fig5

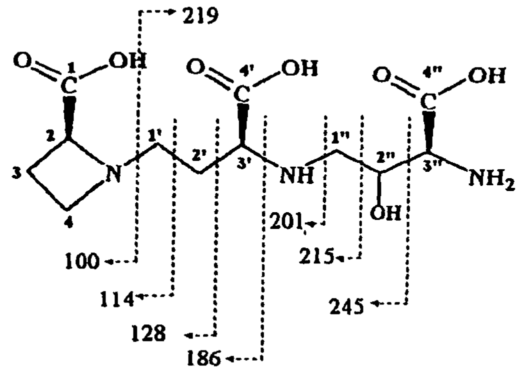

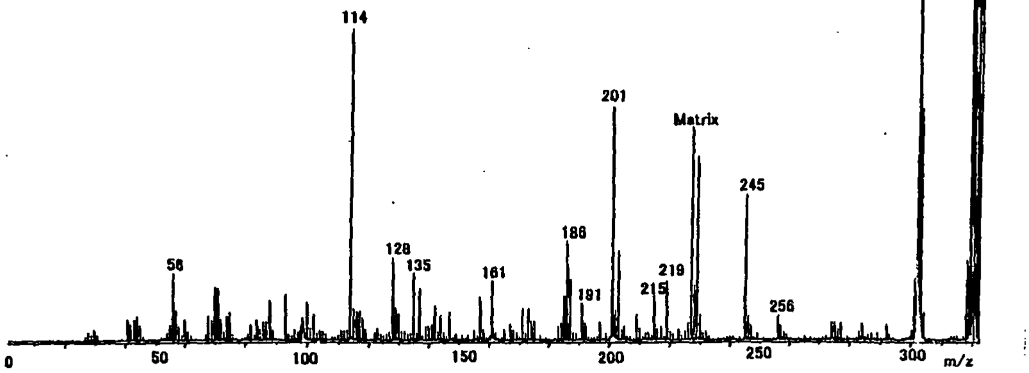
302
M+H
114
201
Matrix
245
186
128
135
58
161
191
215
219
256
0
50
100
150
200
250
300
m/z

## INTERNATIONAL SEARCH REPORT

International application No.
PCT/JP2004/003868

A. CLASSIFICATION OF SUBJECT MATTER
Int.Cl$^7$ C07D205/04

According to International Patent Classification (IPC) or to both national classification and IPC

B. FIELDS SEARCHED

Minimum documentation searched (classification system followed by classification symbols)
Int.Cl$^7$ C07D205/04

Documentation searched other than minimum documentation to the extent that such documents are included in the fields searched

Electronic data base consulted during the international search (name of data base and, where practicable, search terms used)
STN/CAS

C. DOCUMENTS CONSIDERED TO BE RELEVANT

| Category* | Citation of document, with indication, where appropriate, of the relevant passages | Relevant to claim No. |
|---|---|---|
| A | NOMA, M., et al., "A NEW AMINO ACID, NICOTIANAMINE, FROM TOBACCO LEAVES", Tetrahedron Letters, No.22, pages 2017 to 2020, (1971) | 1-12 |
| A | JP 2003-231675 A (Kikkoman Corp.), 19 August, 2003 (19.08.03), (Family: none) | 1-12 |
| A | JP 2002-179647 A (Kikkoman Corp.), 29 June, 2002 (29.06.02), & US 2002/122857 A1 | 1-12 |

☒ Further documents are listed in the continuation of Box C. ☐ See patent family annex.

* Special categories of cited documents:
"A" document defining the general state of the art which is notconsidered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 May, 2004 (14.05.04) | 01 June, 2004 (01.06.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/003868

C (Continuation). DOCUMENTS CONSIDERED TO BE RELEVANT

| Category* | Citation of document, with indication, where appropriate, of the relevant passages | Relevant to claim No. |
|---|---|---|
| A | JP 5-246865 A (Kikkoman Corp.), 24 September, 1993 (24.09.93), (Family: none) | 1-12 |
| A | JP 63-87990 A (Meiji Seika Kaisha, Ltd., et al.), 19 April, 1988 (19.04.88), (Family: none) | 1-12 |
| A | SCHOLZ, G. et al., "STRUCTURE-FUNCTION RELATION SHIPS OF NICOTIANAMINE ANALOGS", Phytochemistry, 27(9) pp 2749-54 (1988) | 1-12 |
| A | RIPPERGER, H. et al., "Peptidanaloga des Phytosi derophors Nicotianamin", Journal fuer Praktische Chemie (Leipzig), 326(2), pp 266-72(1984) | 1-12 |

Form PCT/ISA/210 (continuation of second sheet) (January 2004)